(19) Europäisches Patentamt / European Patent Office / Office européen des brevets

(11) **EP 2 042 877 B1**

(12) **EUROPÄISCHE PATENTSCHRIFT**

(45) Veröffentlichungstag und Bekanntmachung des Hinweises auf die Patenterteilung:
**08.02.2012 Patentblatt 2012/06**

(51) Int Cl.:
*G01P 3/36* *(2006.01)*     *B65H 61/00* *(2006.01)*
*B65H 63/032* *(2006.01)*     *B65H 63/08* *(2006.01)*
*D02G 1/00* *(2006.01)*     *G01N 33/36* *(2006.01)*

(21) Anmeldenummer: **07019133.3**

(22) Anmeldetag: **28.09.2007**

(54) **Verfahren und Vorrichtung zum Messen der Geschwindigkeit eines Garns**

Method and device for measuring the velocity of a thread

Procédé et dispositif destinés à la mesure de la vitesse d'un fil

(84) Benannte Vertragsstaaten:
**BE CH DE FR IT LI**

(43) Veröffentlichungstag der Anmeldung:
**01.04.2009 Patentblatt 2009/14**

(73) Patentinhaber: **Gebrüder Loepfe AG**
**CH-8623 Wetzikon (CH)**

(72) Erfinder:
 • **Pascal, Mauron**
  **8304 Wallisellen (CH)**
 • **Malacarne, Enrico**
  **8630 Rüti (CH)**

(74) Vertreter: **Sutter, Kurt et al**
 **E. Blum & CO. AG**
 **Vorderberg 11**
 **8044 Zürich (CH)**

(56) Entgegenhaltungen:
**EP-A- 0 291 712**     **EP-A- 0 307 644**
**EP-A- 0 329 947**     **EP-A- 0 457 450**
**EP-A- 0 650 915**     **DE-A1- 3 707 552**
**DE-A1- 19 548 256**     **FR-A- 2 720 839**
**FR-A- 2 761 162**     **US-A- 2 950 435**
**US-A- 4 679 932**     **US-A- 5 064 280**
**US-A1- 2004 109 155**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren und eine Vorrichtung zum Messen der Geschwindigkeit eines Garns sowie Verwendungen derselben gemäss Oberbegriff der unabhängigen Ansprüche.

Eine Vorrichtung dieser Art ist aus DE 103 42 383 bekannt. Sie besitzt eine kohärente Lichtquelle, deren Licht in einem Strahlteiler in einen ersten und einen zweiten Lichtstrahl aufgeteilt wird. Diese beiden Lichtstrahlen werden beide auf das Garn geworfen. Weiter ist ein Lichtdetektor vorgesehen, um die Doppler-Interferenz zwischen gestreuten Lichtwellen beider Strahlen zu messen. Entsprechende Sensoren werden als LDA-Sensoren, wobei die Abkürzung LDA für den Begriff "Laser Doppler Anemometrie steht.

Unter Doppler-Interferenz wird dabei die Interferenz zweier Lichtwellen verstanden, deren Frequenzen aufgrund der Reflektion an einem sich bewegenden Objekt leicht unterschiedlich sind. Das Interferenzsignal oszilliert mit einer Frequenz, welche proportional zur Geschwindigkeit des Objekts ist.

Die Vorrichtung gemäss DE 103 42 383 erfordert eine exakte Positionierung des Garns im Kreuzungspunkt der Lichtstrahlen, was apparative Probleme stellt.

Aus US 2004/0109155 ist ein interferometrisches Dopplermessverfahren bekannt, bei welchem ein erster Strahl von einem Objekt zurückgeworfen und zu einem Detektor geführt wird. Zudem wird eine Referenzlichtwelle erzeugt, die ohne Berührung des Objekts direkt zum Detektor geführt wird.

Es ist deshalb Aufgabe der vorliegenden Erfindung, Abhilfe für die genannten apparativen Probleme zu schaffen.

Diese Aufgabe wird vom Gegenstand der unabhängigen Ansprüche gelöst. Anspruchsgemäss ist das Verfahren also dadurch gekennzeichnet, dass einer der Lichtstrahlen am Garn vorbei auf den Lichtdetektor geführt wird. Apparativ wird dies dadurch gelöst, dass dieser Lichtstrahl den für das Garn vorgesehenen Garn-Sollbereich nicht durchsetzt.

Im Gegensatz zu bekannten Geräten zur Garngeschwindigkeitsmessung basierend auf Doppler-Interferenz ist es also nicht notwendig, dass das Garn denjenigen Bereich durchsetzt, in welchem sich die Lichtstrahlen kreuzen. Dies vereinfacht die Justierung des Geräts und dessen Aufbau.

Anspruchsgemäss verlaufen der erste und der zweite Lichtstrahl parallel aber versetzt zueinander. Dies kann mit wenigen, einfachen optischen Komponenten realisiert werden.

Die Erfindung eignet sich besonders für die Überwachung der Garngeschwindigkeit an einer Spulstelle bzw. Umspulmaschine. Sie erlaubt es, die Position und Länge von Fehlstellen, die Länge des gespulten Garns und weitere Parameter des Spulvorgangs exakt zu bestimmen, zu überwachen oder zu steuern.

Weiter kann die Erfindung auch verwendet werden, um die Qualität einer Garnspule zu verbessern oder berührungslos einen Bruch oder ein Einklemmen des Garns zu ermitteln.

Weitere bevorzugte Ausführungen der Erfindung ergeben sich aus den abhängigen Ansprüchen sowie aus der nun folgenden Beschreibung anhand der Figuren. Dabei zeigen:

> Fig. 1 eine erste Ausführung einer Vorrichtung zum Messen der Garngeschwindigkeit (nicht beansprucht),
> Fig. 2 eine zweite Ausführung der Vorrichtung von der Seite,
> Fig. 3 die Ausführung nach Fig. 2 von oben,
> Fig. 4 eine dritte Ausführung der Vorrichtung von der Seite (nicht beansprucht) und
> Fig. 5 die Ausführung nach Fig. 4 von oben (nicht beansprucht).

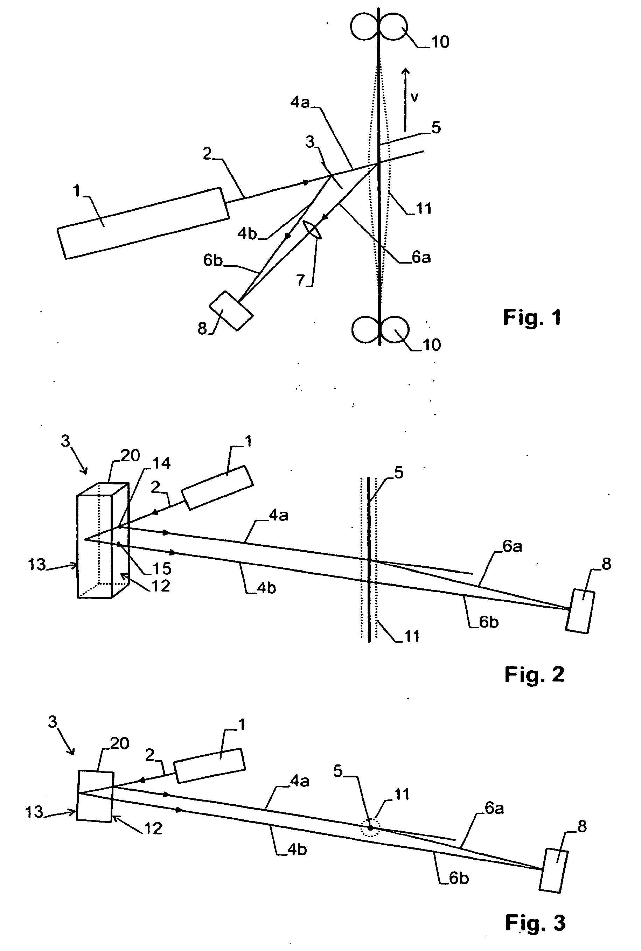

Die in Fig. 1 dargestellte Ausführung eines LDA-Sensors besitzt eine kohärente Lichtquelle 1, vorzugsweise einen Halbleiter-Laser, welcher einen Lichtstrahl 2 (im Folgenden der "ursprüngliche Lichtstrahl" genannt) erzeugt. Der ursprüngliche Lichtstrahl 2 tritt auf einen Strahlteiler 3, von welchem er in einen ersten Lichtstrahl 4a und einen zweiten Lichtstrahl 4b aufgeteilt wird. Vorzugsweise wird der grösste Teil der Lichtleistung (z.B. 90%) in den ersten Lichtstrahl gekoppelt, da nur ein kleiner Teil seines Lichts später auf den Detektor fallen wird.

Der erste Lichtstrahl 4a trifft auf ein mit einer Geschwindigkeit v laufendes Garn 5, wo er gestreut wird. Ein Teil 6a der gestreuten Lichtwellen (im Folgenden die "ersten Lichtwellen") genannt, tritt durch eine optionale Abbildungsoptik 7 und wird auf einen Detektor 8 geworfen. Gleichzeitig wird auch mindestens ein Teil 6b des zweiten Lichtstrahls (im Folgenden die "zweiten Lichtwellen" genannt) auf den Detektor 8 geführt.

Der Wegunterschied zwischen den ersten und zweiten Lichtwellen ist so gewählt, dass er kleiner ist als die Kohärenzlänge des von der Lichtquelle 1 erzeugten Lichts, so dass die Lichtwellen am Ort des Detektors 8 interferieren. Zudem ist die Geometrie der Anordnung so gewählt, dass nur solche erste Lichtwellen 6a auf den De-tektor 8 treffen, die am Garn 5 so gestreut (d.h. so reflektiert oder so gebrochen) wurden, dass ihre Frequenz bei bewegten Garnen einer Dopplerverschiebung unterliegt, d.h. dass der Impuls der Photonen entlang der Laufrichtung des Garns 5 bei der Streuung geändert wurde. Die Frequenzverschiebung ist von der Geschwindigkeit des Garns aber auch vom Streuwinkel abhängig.

Somit kann am Detektor 8 eine Doppler-Interferenz beobachtet werden, d.h. die gemessene Intensität oszilliert mit einer Frequenz proportional zur Geschwindigkeit des Garns 5. Es ist darauf zu achten, dass nur ein enger Bereich von Streuwinkeln den Detektor erreicht, um ein Ausschmieren der gemessenen Frequenz zu vermeiden. Der Winkel zwischen den Strahlen 6a und 6b ist klein genug zu wählen, um ein räumliches Interferenzmuster auf dem Detektor zu vermeiden. Alternativ kann der Detektor so dimensioniert werden, dass er deutlich kleiner als die Wellenlänge des Interferenzmusters ist.

Wie aus Fig. 1 ersichtlich, wird also der erste Lichtstrahl 4a auf das Garn 5 geworfen, während der zweite Lichtstrahl 4b ohne Berührung des Garns an diesem vorbei auf den Lichtdetektor 8 geführt wird. Um dies sicherzustellen, können Garnführungsmittel 10 vorgesehen sein, welche z.B. als Öffnungen und/oder Führungsrollen am Anfang und Ende der Messvorrichtung ausgestaltet sein können, welche das Garn 5 so führen, dass es in einem vorgegebenen Garn-Sollbereich 11 bleibt. Solche Garnführungsmittel können auch verwendet werden, um unerwünschte, transversale Bewegungen des Garns zu unterdrücken, welche die Messung der longitudinalen Garngeschwindigkeit unerwünscht beeinflussen könnten. Der erste Lichtstrahl 4a durchsetzt den Garn-Sollbereich 11, während der zweite Lichtstrahl 4b diesen nicht durchsetzt.

Die Abbildungsoptik 7 ist, wie erwähnt, optional. Sie bildet den Bereich des Garns, welcher vom ersten Lichtstrahl 4a getroffen wird, auf den Detektor 8 oder (äquivalent) auf eine Blende vor dem Detektor 8 ab. Dadurch wird einerseits sichergestellt, dass nur solche Lichtwellen auf den Detektor fallen, welche bei einer Streuung des ersten Lichtstrahls 4a am Garn 5 entstanden sind, und andererseits kann die Intensität und Gesamtleistung der beim Detektor 8 ankommenden Wellen gegenüber der gleichen Anordnung ohne Abbildungsoptik 7 erhöht werden.

Da der zweite Lichtstrahl 4b nicht aus dem von der Abbildungsoptik 7 auf den Detektor 8 abgebildeten Bereich stammt, wird er vorzugsweise an der Abbildungsoptik vorbei direkt auf den Lichtdetektor 8 geführt.

Fig. 2 und 3 zeigen eine zweite Ausführung der erfindungsgemässen Vorrichtung, bei welcher die Strahlteiler-Anordnung 3 nicht von einem einfachen Strahlteiler sondern von einem etwas anders ausgestalteten, mindestens teilweise transparenten Körper 20 gebildet wird. In der gezeigten Ausführung handelt es sich bei der Strahlteiler-Anordnung 3 um eine einige Millimeter dicke Platte mit zwei parallelen Oberflächen 12, 13, von welcher das Licht des ursprünglichen Lichtstrahls 2 durch Reflexion an Vorder- bzw. Rückseite in den ersten und den zweiten Strahl 4a bzw. 4b aufgeteilt wird.

Hierzu wird der ursprüngliche Lichtstrahl 2 in einem Punkt 14 auf die erste Oberfläche 12 geworfen, von wo ein Teil als erster Lichtstrahl 4a reflektiert wird. Das restliche Licht durchtritt sodann den Körper 20 um an der gegenüberliegenden zweiten Oberfläche 13 reflektiert zu werden. Es tritt an einem Punkt 15 durch die erste Oberfläche 12 aus dem Körper 20 des Strahlteilers 3 aus und bildet den zweiten Lichtstrahl 4b.

Da die Oberfläche 12 und 13 parallel zueinander angeordnet sind, verlaufen auch die Lichtstrahlen 4a und 4b parallel zueinander. Da der ursprüngliche Lichtstrahl 2 jedoch schräg auf die erste Oberfläche 12 auftritt, sind sie zueinander versetzt. Sie verlaufen so, dass nur der erste Lichtstrahl 4a auf das Garn 5 trifft, während der zweite Lichtstrahl 4b am Garn-Sollbereich 11 vorbei geführt wird und direkt auf den Detektor 8 trifft. Zudem treffen auf den Detektor 8 erste Lichtwellen 6a des ersten Lichtstrahls 4a auf, welche am Garn 5 gestreut wurden. Dabei kommt es wiederum zu einer Doppler-Interferenz, welche vom Detektor 8 gemessen werden kann.

Die relative Leistung bzw. Intensität der beiden Lichtstrahlen 4a, 4b kann durch geeignete Beschichtung der Oberflächen 12 und 13 der Strahlteiler-Anordnung 3 eingestellt werden. Insbesondere ist die erste Oberfläche 12 im Bereich des Punkts 14 relativ stark reflektierend und am zweiten Punkt 15 möglichst tief reflektierend ausgestaltet, während die zweiten Oberfläche 13 mit einer hoch reflektierenden Beschichtung versehen ist.

Es ist auch denkbar, den am Punkt 14 reflektierten Strahl als zweiten Lichtstrahl 4b zu verwenden und jenen, der am Punkt 15 austritt, als ersten Lichtstrahl 4a. Die Beschichtung der Oberfläche 12 kann in diesem Fall überall gering reflektierend sein.

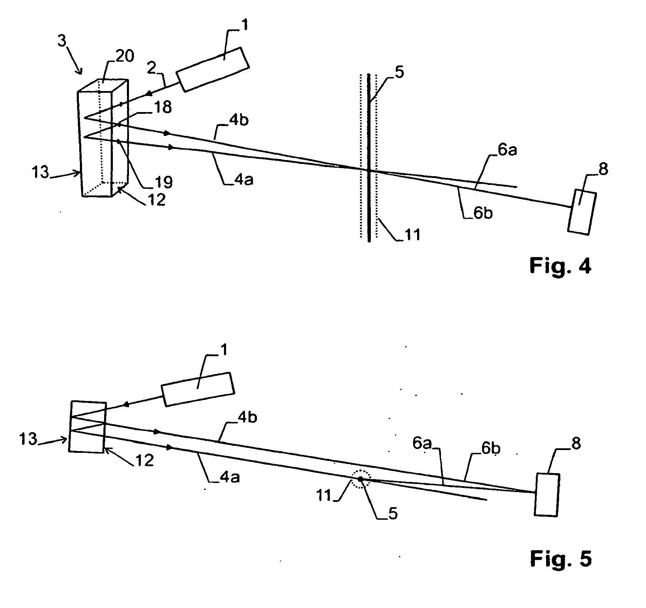

Eine weitere Ausführung mit einem keilförmigen Körper 20 als Strahlteiler-Anordnung 3 ist in Fig. 4 und 5 dargestellt. Hier sind die Oberflächen 13 und 14 nicht parallel, sondern verlaufen unter einem Winkel von einigen Grad zueinander. Der ursprüngliche Strahl tritt zuerst durch die erste Oberfläche 12 in den Körper 20 ein, wird von der zweiten Oberfläche 13 reflektiert und tritt am Punkt 18 teilweise aus dem Körper 20 aus, um den zweiten Lichtstrahl 4b zu erzeugen. Der Rest des Strahls wird wieder in den Körper 20 zurückgeworfen, um nochmals an der Oberfläche 13 reflektiert zu werden und an einem Punkt 19 der ersten Oberfläche 12 als erster Lichtstrahl 4a auszutreten. Auch hier verlaufen der erste und der zweite Lichtstrahl 4a bzw. 4b versetzt, sie laufen jedoch nicht parallel, sondern schräg zueinander. Der erste Lichtstrahl 4a trifft auf das Garn 5, von wo die erste Lichtwelle 6a zum Detektor 8 geführt wird, während der zweite Lichtstrahl 4b am Garn-Sollbereich 11 vorbei direkt als zweite Welle 6b zum Detektor 8 gelangt.

In den Figuren zu allen Ausführungen sind die Lichtstrahlen als Striche gezeichnet. Vorzugsweise ist aber zumindest der erste Strahl 4a im Bereich des Garns 5 aufgeweitet und durchsetzt den Garn-Sollbereich 11 auf dessen ganzer Breite, so dass das Garn 5 unabhängig von seiner momentanen Position im Garn-Sollbereich dauernd beleuchtet bleibt.

Vorzugsweise ist zumindest der erste Lichtstrahl im Bereich des Garns unfokussiert oder nur schwach fokussiert und besitzt einen Durchmesser von mindestens 1 mm, insbesondere von mindestens 2 mm, so dass das Garn bei allfälligen Bewegungen nicht aus dem Strahl heraustritt.

Die Erzeugung des ersten und des zweiten Lichtstrahls 4a bzw. 4b durch Reflexionen an einem einzigen, zumindest teilweise transparenten Körper 20 hat den Vorteil, dass die Anordnung verhältnismässig unempfindlich ist gegenüber Vibrationen der Strahlteiler-Anordnung, da diese beide Lichtstrahlen 4a, 4b in gleicher Weise beeinflussen und so nicht zu zusätzlichen, störenden Gangunterschieden führen.

Die beschriebene Vorrichtung kann in verschiedenster Weise modifiziert werden. Mit Vorteil werden der erste und der zweite Lichtstrahl jedoch so geführt, dass sie sich möglichst nicht überlappen.

Die Vorrichtung wird vorzugsweise in einer Spulmaschine eingesetzt, wie sie z.B. in DE 103 42 383 beschrieben ist. Sie erlaubt es, die Geschwindigkeit und, mittels Integration der Geschwindigkeit über die Zeit, die gespulte Länge des Garns festzustellen.

Insbesondere kann sie verwendet werden, um die Position und/oder Länge von Fehlstellen zu bestimmen.

Zur Messung der Position und/oder Länge der Fehlstellen und/oder zur Messung der gespulten Garnlänge werden die gemessenen Geschwindigkeitswerte integriert. Dies entspricht z.B. einer Summation aller zeitdiskret erfassten Geschwindigkeitswerte jeweils multipliziert mit dem Zeitintervall, das dem jeweiligen Geschwindigkeitswert zugeordnet ist.

In vielen Fällen kann es von Vorteil sein, aus der gemessenen Garngeschwindigkeit auch die Beschleunigung des Garns zu ermitteln, da diese oft ein direktes Mass für die auf das Garn wirkende Zugkraft ist. Die Beschleunigung kann als Mass für die (Netto-) Garnzugkraft ausgegeben oder in anderer Weise verwendet werden. Ist die Beschleunigung zu gross, so kann ein Warnsignal ausgegeben und/oder die auf das Garn wirkende Zugkraft reduziert und/oder die Spulgeschwindigkeit reduziert und/oder ein Garnabzugbeschleuniger verstellt und/oder der Spulprozess beendet werden.

Falls das Garn nach der Messung aufgespult wird, ist die Vorrichtung auch dafür geeignet, die totale Länge des aufgespulten Garns zu ermitteln, ebenfalls durch Integration des Geschwindigkeitssignals.

Sie ist auch dafür geeignet, einen Schlupf des Garns zu bestimmen, indem die Abweichung der gemessenen Länge oder Geschwindigkeit von einer erwateten Länge oder Geschwindigkeit ermittelt wird.

Wird das Garn in einer Spulstelle durch eine Nutentrommel geführt, wie in DE 103 42 383 beschrieben, so kann die korrekte Führung des Garns in der Trommel überwacht werden. Ist das Garn korrekt geführt, so zwingt die Nutentrommel dem Garn einen von der Geometrie der Trommelnuten vorgegebenen, vorbekannten Verlauf auf. Wird der Verlauf der Geschwindigkeit des Garns mit dem vorbekannten Verlauf verglichen, so kann bei ausreichend starken Abweichungen darauf geschlossen werden, dass das Garn nicht mehr korrekt geführt ist.

Generell kann der Geschwindigkeitsverlauf des Garns auch zu anderen Zwecken ausgewertet werden, z.B. zur Qualitätsüberwachung beim Spulprozess, siehe unten.

Verallgemeinert gesagt, können die von der Vorrichtung ermittelten Messwerte auch dazu verwendet werden, den Spulprozess zu überwachen oder zu steuern, z.B. auch indem der Spulprozess unterbrochen wird, wenn der Verlauf der Geschwindigkeit nicht dem erwarteten Verlauf entspricht oder indem die Länge und Position von Fehlstellen vorgegebene Grenzwerte oder Vorgaben überschreiten.

Das vorliegende Verfahren bzw. die vorliegende Vorrichtung kann insbesondere auch für folgende Zwecke verwendet werden:

1. Erfassen und/oder Verbessern der Garnqualität an einer Spulstelle einer Textilmaschine.

In diesem Fall wird die beschriebene Vorrichtung an der Spulstelle angebracht und dient dazu, die Garnqualität des laufenden Garns zu erfassen. Hierzu können insbesondere die folgenden Massnahmen vorgesehen sein:

- Die zu diskreten Zeiten ermittelten Geschwindigkeitswerte werden in der oben erwähnten Weise integriert, um die Länge und/oder Position von Fehlstellen zu ermitteln, ggf. in Kombination mit Daten von anderen Sensoren. Diese Information dient der Klassierung der Fehlstellen und auch zu deren räumlicher Ortung. Insbesondere erlaubt sie es auch, das Garn zum Herausschneiden einer Fehlstelle ortsgenau zurückzuspulen, indem das Zurückspulen abhängig von der Position und/oder Länge der Fehlstelle erfolgt.
- Es kann die Haarigkeit des laufenden Garns zur Bestimmung der Garnqualität erfasst werden, da die Streuung, welche zur Bildung der ersten Lichtwellen 6a führt, oft auf die Haarigkeit des Garns zurückzuführen ist. Bei einem stark haarigen Garn ist die Streuung stark. Zudem führt jedes Haar in der Regel zu einer kurzzeitig ansteigenden Streuung und so zu einem individuellen Burst-Paket, welches erfasst werden kann. Somit kann aus der Stärke der vom Detektor erfassten Dopplerinterferenz auf die Haarigkeit geschlossen werden. Beispielsweise ist die zeitgemittelte Amplitude des Doppler-Interferenzsignals ein Mass für die mittlere Haarigkeit, oder es kann die Zahl der Burst-Pakete pro Zeiteinheit ermittelt werden, welche jeweils eine vorgegebene Amplitude überschreiten, und daraus ein Mass für die Zahl der Haare pro Zeiteinheit abgeschätzt werden. Das so ermittelte Mass der Haarigkeit kann in geeigneter Weise angezeigt bzw. ausgegeben werden. Bei Überschreiten einer vorgegebenen Grenzhaarigkeit kann ein Warnsignal ausgegeben und/oder die Spulengeschwindigkeit reduziert und/oder die Garnzugkraft reduziert und/oder der Spulprozess beendet werden.

## 2. Erfassen und/oder Verbessern der der Qualität einer Garnspule.

Auch in diesem Fall wird die beschriebene Vorrichtung an einer Spulstelle angebracht und dient dazu, die Qualität der Spule zu erfassen, die durch Aufspulen eines Garns erhalten wird, wobei die Vorrichtung das Garn beim Aufspulen erfasst. In diesem Zusammenhang können insbesondere die folgenden Massnahmen vorgesehen sein:

- Die zu diskreten Zeiten ermittelten Geschwindigkeitswerte werden in der oben erwähnten Weise integriert, um die Länge des aufgespulten Garns zu ermitteln, und/oder der Spulprozess wird beim Erreichen einer vorgegebenen Solllänge beendet und/oder es wird ein Spulenwechselsignal ausgegeben und/oder ein Spulenwechselaggregat wird aktiviert.
- Durch Messung des Geschwindigkeitsverlaufs in der oben erwähnten Weise kann überwacht werden, dass das Garn richtig in der Nutentrommel geführt und die Spule richtig aufgewickelt wird. Insbesondere kann der Geschwindigkeitsverlauf auch einer Spektralzerlegung unterworfen werden, z.B. durch Fourier-Transformation. Das so erhaltene Frequenzprofil kann mit einem unteren und/oder oberen Grenz-Frequenzprofil verglichen werden, wobei bei Überschreiten des oberen Grenz-Frequenzprofils bzw. Unterschreiten des unteren Grenz-Frequenzprofils ein Fehler angezeigt wird oder Massnahmen zur Korrektur des Spulprozesses ergriffen werden (z.B. indem die Spulgeschwindigkeit reduziert wird).
- Ausserdem können die Messsignale vorteilhaft auch bezüglich eines oder mehrerer Durchmesser der Spule ausgewertet werden. Treibt z.B. eine zylindrische Trommel eine konische Spule an, so ergibt sich auf den Spulenhub gesehen nur eine sehr begrenzte Zone, in der die Umfangsgeschwindigkeiten der Trommel mit derer der Spule identisch sind. Diese neutrale Zone wird auch als treibender oder antreibender Durchmesser bezeichnet.
  Dieser Durchmesser wird konventionell über das Verhältnis von Spulendrehzahl zu Trommeldrehzahl berechnet. Nachteilig dabei ist, dass bei durchmesserabgestellten Spulpartien, bei denen die Spulpartie bei Erreichen eines bestimmten Durchmessers beendet wird, im Falle einer konischen Spule dieser antreibende Durchmesser als Abbruchkriterium für den Spulprozess verwendet wird. Für den Endabnehmer des Produkts Spulen ist jedoch nicht dieser Durchmesser von Belang, sondern oft der Konus-Aussendurchmesser. Weiterhin muss bedacht werden, dass der antreibende Durchmesser, abhängig von den Spulbedingungen, sich an unterschiedlichen Positionen befinden und irgendwo auf dem Spulenhub zwischen kleinem und grossem Durchmesser liegen kann.
  Mit dem erfindungsgemässen Verfahren kann demgegenüber auch an den Umkehrpunkten der Fadenchangierung direkt der Aussendurchmesser und/oder der Innendurchmesser einer konischen Spule berechnet werden. Dabei ergeben sich die folgenden Zusammenhänge:

$$v_{Aussen} = r_{Aussen} \cdot \omega_{Spule}$$

und

$$v_{Innen} = r_{Innen} \cdot \omega_{Spule}$$

wobei r der jeweilige (kleine oder grosse) Spulendurchmesser, v die jeweilige Umfangsgeschwindigkeit und $\omega_{Spule}$ die Winkelgeschwindigkeit der Spule sind.

Wenn man von konstanter Winkelgeschwindigkeit ausgeht und annimmt, dass am Innen- und Aussenradius die Fadengeschwindigkeiten identisch mit den Umlaufgeschwindigkeiten der Spule sind (da in den Umkehrpunkten des Verlegedreiecks lediglich eine sehr geringe oder gar keine Horizontalgeschwindigkeit und somit zumindest annähernd nur eine Tangentialgeschwindigkeit vorliegt), so ergibt sich der Zusammenhang

$$\frac{v_{Aussen}}{v_{Innen}} = \frac{r_{Aussen}}{r_{Innen}}$$

Wird gleichzeitig mit der Erfassung der Spulengeschwindigkeit auch die Spulendrehzahl bzw. $\omega_{Spule}$ erfasst, was vorzugsweise über einen an der Spule angeordneten Impulsgeber möglich ist, kann so unter Berücksichtigung des Verlegehubs auch der Aussen- bzw. Innendurchmesser der konischen Spule sehr genau bestimmt werden.

Dabei ist es weiter von Vorteil, dass der Spulprozess nicht nur bei Erreichen eines bestimmten Durchmessers abstellbar ist, sondern dass auch die exakte Lage des antreibenden Durchmessers ermittelt und ausgegeben werden kann.

Mit den gleichen Überlegungen kann nicht nur der Durchmesser einer konischen Spule, sondern auch jener einer zylindrischen Spule aus der Garngeschwindigkeit unter Berücksichtigung des Verlegehubs ermittelt werden.

- Weiter können die Messsignale auch zur Bestimmung der Dichte der Spule eingesetzt werden. Diese Dichte, ermittelt aus der Garnfeinheit, der Spulengeometrie und der gespulten Garnlänge, ist ein Qualitätsmerkmal des gespulten Gutes. Ist beispielsweise die Spulendichte in einem statistisch vergleichbaren Kollektiv stark abweichend, so kann davon ausgegangen werden, dass die vom Kollektiv abweichenden Spulen infolge einer zu hohen Fadenspannung und/oder eines zu hohen Anpressdrucks durch den Spulenrahmen oder infolge anderer Umstände als qualitätsmindernd zu bezeichnen sind. Beispielsweise kann aus den in der oben beschriebenen Weise ermittelten Spulendurchmessern das Volumen der Spule ermittelt werden, wobei der Quotient zwischen Länge des aufgespulten Garns und Volumen der Spule eine für die Garnfeinheit typische, vorgegebene Dichte ergeben sollte. Es kann also ein oberer und/oder unterer Soll-Dichtewert vorgegeben werden, mit welchem der ermittelte Dichtewert verglichen wird. Bei Über- bzw. Unterschreiten des Soll-Dichtewerts kann ein Warnsignal ausgegeben werden.

- Weiter kann auch der Schlupf zwischen der Trommel und der Spule bezüglich eines Auftrags von Paraffin auf den Faden ausgewertet werden. Nutzt man zur exakten Durchmesserbestimmung über die vorangehend beschriebene Ermittlung der Geschwindigkeiten an den Umkehrpunkten die von der Spulenachse abgenommene Spulengeschwindigkeit, so kann der treibende Durchmesser der konischen Spule dynamisch, also während des Spulprozesses, erfasst werden. Aufgrund von Beschleunigungsmechanismen (Bildstörung), die während des Spulbetriebs auf die Spule wirken, wird die Position des antreibenden Durchmessers im Falle der Paraffinierung stark wandern, während ohne Paraffinauftrag die Position relativ konstant bleibt. Vorteilhafterweise kann ein Warnsignal ausgegeben und/oder der Spulprozess automatisch beendet werden, wenn der Paraffinauftrag von einem vorgegebenen Zielwert abweicht oder einen vorgegebenen Grenzwert unterschreitet. Dabei wird der Spulprozess automatisch korrigiert oder beendet, wenn der Schlupf von einem Soll-Schlupf oder vorgegebenen Schlupfprofil abweicht.
- Ferner kann der Schlupf zwischen Trommel und Spule beim Hochlaufen des Spulprozesses überwacht werden. Dabei wird der Antrieb der Trommel vorzugsweise auf einen vorgegebenen Soll-Schlupf oder ein vorgegebenes Schlupfprofil geregelt, oder der Spulprozess wird automatisch korrigiert oder beendet, wenn der Schlupf vom Soll-Schlupf oder Schlupfprofil abweicht.

Der Spulprozess ist ein diskontinuierlicher Prozess, der aufgrund der Kopsvorlagen mehrmals (abhängig vom Material und Spulendurchmesser mehr als 40-mal) unterbrochen wird. Die beschriebene Überwachung des Schlupfes der Trommel erlaubt ein sehr schnelles Hochfahren der Spule.

Durch den Sensor kann über die Erfassung der exakten Spulgeschwindigkeit mit gleichzeitiger Erfassung der Trommeldrehzahl der Schlupf zwischen Antriebstrommel und Spule exakt bestimmt und damit über den Trommelantrieb eingestellt bzw. geregelt werden, so dass eine hohe Prozessgeschwindigkeit bei hoher Produktionsqualität erreicht werden kann.

### 3. Bestimmen eines Bruchs eines laufenden Fadens oder eines Einklemmens eines laufenden Fadens

Gemäss einer weiteren Anwendung der Erfindung kann mit der beschriebenen Technik der Bruch eines laufenden Fadens oder ein Einklemmen desselben berührungslos ermittelt werden, insbesondere (aber nicht nur) im Spulprozess.

Die Qualität der Spulenproduktion kann hinsichtlich eines möglichen Bruchs des laufenden Garns und/oder eines möglichen Einklemmens des laufenden Fadens zwischen der Spule, auf die der Faden aufgewickelt wird, und einer die Spule antreibenden Antriebswalze, überwacht und gegebenenfalls optimiert werden, indem mit der hier beschriebenen Technik die Garngeschwindigkeit gemessen wird.

Neben der Spulung mit Hilfe einer Verlegeund Antriebswalze (Nutentrommel) existieren weitere Spultechnologien. Diese benutzen eine Antriebs- oder Stützwalze im Bereich des Spulenantriebs, wobei der Antrieb also direkt über die Spulenachse oder über die Antriebswalze erfolgt. Die Fadenverlegung wird hierbei über ein separates Aggregat realisiert.

Dabei kann während des Spulprozesses aufgrund der Fadenbelastung ein Fadenbruch im Klemmpunkt zwischen Antriebswalze und Spule auftreten. In solchen Fällen besteht die Gefahr, dass der Faden trotz des Bruchs weiter transportiert und aufgrund der vorhandenen Konvektion durch die Spule mitgerissen wird. Eine andere Möglichkeit ist, dass der Faden über den Klemmpunkt nach hinten wegbefördert wird und irgendwo an einem Prozessaggregat hängen bleibt. Beide Fälle sind unerwünscht und beeinträchtigen die Spulenqualität bzw. den Spulprozess signifikant.

Über die Geschwindigkeitsmessung mittels eines LDA-Sensors kann vorteilhafterweise ein Garnbruch frühzeitig detektiert werden. Es kann dabei davon ausgegangen werden, dass der Faden unmittelbar nach dem Bruch eine kurze Geschwindigkeitsänderung erfährt, insbesondere eine Reduzierung (falls der Bruch hinter dem LDA-Sensor stattfindet). Diese Geschwindigkeitsänderung kann mittels Auswerten der Signale des Sensors ermittelt werden.

### Patentansprüche

1. Verfahren zum Messen der Geschwindigkeit eines Garns (5), bei welchem eine erste und eine zweite kohärente Lichtwelle (6a, 6b) zur Interferenz gebracht werden, wobei die erste Lichtwelle (6a) vom Garn (5) gestreutes Licht enthält und von einem ersten Lichtstrahl (4a) stammt, der auf das Garn (5) geworfen wird, und wobei die zweite Lichtwelle (6b) von einem zweiten Lichtstrahl (4b) stammt, und wobei die Geschwindigkeit des Garns (5) aus der Doppler-Interferenz zwischen den Lichtwellen (6a, 6b) in einem Lichtdetektor (8) ermittelt wird, **dadurch gekennzeichnet, dass** der zweite Lichtstrahl (4b) ohne Berühren des Garns (5) direkt als zweite Lichtwelle (6b) auf den Lichtdetektor (8) geführt wird, wobei der erste und der zweite Lichtstrahl (4a, 4b) parallel aber versetzt zueinander verlaufen.

2. Verfahren nach Anspruch 1, wobei der erste und der zweite Lichtstrahl (4a, 4b) sich nicht überlappen.

3. Verfahren nach einem der vorangehenden Ansprüche, wobei der erste und der zweite Lichtstrahl (4b) erzeugt werden, indem ein ursprünglicher Lichtstrahl (2) von einer kohärenten Lichtquelle auf einen mindestens teilweise transparenten Körper (20) geworfen und von diesem Körper (20) durch Reflektionen aufgeteilt wird.

4. Verfahren nach Anspruch 3, wobei der erste und der zweite Lichtstrahl (4a, 4b) erzeugt werden, indem ein ursprünglicher Lichtstrahl (2) von einer kohärenten Lichtquelle (1) auf einen mindestens teilweise transparenten Körper (20) geworfen und von diesem Körper (20) durch Reflektionen aufgeteilt wird, wobei die Reflektionen an zueinander parallelen Oberflächen (12, 13) des Körpers (20) stattfinden.

5. Verfahren nach Anspruch 4, wobei der ursprüngliche Lichtstrahl (2) durch eine erste Oberfläche (12) in den Körper (20) eintritt und dabei ein Teil des ursprünglichen Lichtstrahls (2) zum Erzeugen des ersten oder zweiten Lichtstrahls (4a, 4b) reflektiert wird, wobei sodann der ursprüngliche Lichtstrahl (2) an einer der ersten Oberfläche (12) gegenüberliegenden zweiten Oberfläche (13) des Körpers (20) reflektiert wird und sodann wieder durch die erste Oberfläche (12) aus dem.Körper (20) austritt zum Erzeugen des zweiten bzw. ersten Lichtstrahls (4a, 4b).

6. Verfahren nach einem der vorangehenden Ansprüche, wobei ein Bereich des Garns (5) mit einer Abbildungsoptik (7) auf den Lichtdetektor (8) abgebildet wird und wobei der zweite Lichtstrahl (4b) an der Abbildungsoptik (7) vorbei auf den Lichtdetektor (8) geführt wird.

7. Verfahren nach einem der vorangehenden Ansprüche, wobei aus der gemessenen Geschwindigkeit Position und/oder Länge von Fehlstellen im Garn ermittelt werden.

8. Verfahren nach einem der vorangehenden Ansprüche, wobei das Garn aufgespult wird und wobei aus der gemessenen Geschwindigkeit eine Länge des aufgespulten Garns bestimmt wird.

9. Verfahren nach einem der vorangehenden Ansprüche, wobei das Garn aufgespult wird und wobei aus der gemessenen Geschwindigkeit ein zeitabhängiger Geschwindigkeitsverlauf aufgenommen wird.

10. Verfahren nach Anspruch 9, wobei das Garn in einer Spulstation durch eine Nutentrommel geführt wird, welche dem Garn einen von der Geometrie der Nutentrommel vorgegebenen, vorbekannten Verlauf aufzwingt, und wobei der aufgenommene Geschwindigkeitsverlauf mit dem vorbekannten Verlauf verglichen wird, insbesondere um zu prüfen, ob das Garn korrekt in der Nutentrommel geführt wird.

11. Verfahren nach einem der vorangehenden Ansprüche, wobei aus den gemessenen Geschwindigkeiten eine Beschleunigung des Garns ermittelt wird, und insbesondere dass die Beschleunigung als Mass für eine Garnzugkraft verwendet wird.

12. Verfahren nach einem der vorangehenden Ansprüche, wobei der erste Lichtstrahl (4a) beim Garn unfokussiert ist und/oder einen Durchmesser von mindestens 1 mm, insbesondere mindestens 2 mm aufweist.

13. Vorrichtung zum Messen der Geschwindigkeit eines Garns (5), umfassend
Garnführungsmittel (10) zum Führen des Garns (5) durch einen Garn-Sollbereich (11),
eine kohärente Lichtquelle (1) zum Erzeugen eines ursprünglichen Lichtstrahls (2),
eine Strahlteiler-Anordnung (3) zum Erzeugen eines ersten und eines zweiten Lichtstrahls (4b) aus dem ursprünglichen Lichtstrahls (2), wobei die Strahlteiler-Anordnung so ausgestaltet ist, dass der erste Lichtstrahl (4a) den Garn-Sollbereich durchsetzt um dort eine am Garn (5) gestreute erste Lichtwelle (6a) zu erzeugen, und
einen Lichtdetektor (8) zum Messen einer Doppler-Interferenz zwischen der ersten Lichtwelle (6a) und einer aus dem zweiten Lichtstrahl (4b) erzeugten zweiten Lichtwelle (6b),
wobei die Strahlteiler-Anordnung (3) so ausgestaltet ist, dass der zweite Lichtstrahl (4b) den Garn-Sollbereich nicht durchsetzt und somit ohne Berühren des Garns (5) auf den Lichtdetektor (8) trifft
**dadurch gekennzeichnet, dass** die Vorrichtung so ausgebildet ist, dass der zweite Lichtstrahl (4b) als zweite Lichtwelle (6b) direkt auf den Lichtdetektor (8) trifft, und dass die Strahlteiler-Anordnung (3) einen transparenten Körper (20) mit einer ersten und einer zweiten Oberfläche (12, 13) aufweist, welche parallel zueinander verlaufen, wobei der ursprüngliche Lichtstrahl (2) durch Reflektionen an der ersten und der zweiten Oberfläche (12, 13) in den ersten und den zweiten Lichtstrahl (4a, 4b) aufgeteilt wird, und wobei der erste und der zweite Lichtstrahl (4a, 4b) parallel und versetzt zueinander verlaufen.

14. Vorrichtung nach Anspruch 13, wobei eine Abbildungsoptik (7) vorgesehen ist um einen Bereich des Garns (5) auf den Lichtdetektor (8) abzubilden und wobei der zweite Lichtstrahl (4b) an der Abbildungsoptik (7) vorbei auf den Lichtdetektor (8) geführt ist.

15. Verwendung des Verfahrens oder der Vorrichtung nach einem der vorangehenden Ansprüche zum Erfassen und/oder Verbessern der Garnqualität eines laufenden Garns an einer Spulstelle einer Textilmaschine.

16. Verwendung nach Anspruch 15, wobei die Position und/oder Länge einer Fehlstelle durch die Dopplerinterferenz erfasst wird und insbesondere wobei zum Herausschneiden einer Fehlstelle das Garn abhängig von der Position und/oder Länge der Fehlstelle zurückgespult wird.

17. Verwendung nach einem der Ansprüche 15 oder 16, wobei aus der Dopplerinterferenz ein Mass für die Haarigkeit des Garns ermittelt wird, und insbesondere wobei dieses Mass ausgegeben wird und/oder mit einer Grenzhaarigkeit verglichen wird um bei Überschreiten der Grenzhaarigkeit ein Warnsignal auszugeben und/oder eine Spulengeschwindigkeit zu reduzieren und/oder eine Garnzugkraft zu reduzieren und/oder einen Spulprozess zu beenden.

18. Verwendung des Verfahrens oder der Vorrichtung nach einem der Ansprüche 1 bis 14 zum Erfassen und/oder Verbessern der Qualität einer Garnspule, die durch Aufwickeln des Garns erhalten wird.

19. Verwendung nach Anspruch 18, wobei eine aufgespulte Länge ermittelt wird und/oder bei Erreichen einer vorgegebenen Solllänge der Spulprozess beendet oder ein Spulwechsel eingeleitet wird.

20. Verwendung nach einem der Ansprüche 18 oder 19, wobei ein Geschwindigkeitsverlauf des Garns gemessen und der Geschwindigkeitsverlauf einer Spektralzerlegung unterworfen wird um ein Frequenzprofil zu erhalten, welches mit einem oberen und/oder einem unteren Grenz-Frequenzprofil verglichen wird, wobei bei Überschreiten des oberen Grenz-Frequenzprofils bzw. unterschreiten des unteren Grenz-Frequenzprofils ein Fehler angezeigt wird oder Massnahmen zur Korrektur des Spulprozesses ergriffen werden.

21. Verwendung nach einem der Ansprüche 18 bis 20, wobei aus der gemessenen Geschwindigkeit unter Berücksichtigung des Verlegehubs ein Durchmesser der Spule, insbesondere ein innerer und/oder ein äusserer Durchmesser einer konischen Spule, ermittelt wird, und insbesondere wobei die Spulendrehzahl ermittelt und aus der Spulendrehzahl zusammen mit der gemessenen Geschwindigkeit des Garns der Durchmesser ermittelt wird.

22. Verwendung nach einem der Ansprüche 18 bis 21, wobei aus der gemessenen Geschwindigkeit eine Dichte der Spule ermittelt wird, und insbesondere wobei die Dichte der Spule ausgegeben wird und oder ein Warnsignal ausgegeben wird, wenn die Dichte einen vorgegebenen oberen und/oder unteren Soll-Dichtewert unter- oder überschreitet.

23. Verwendung nach einem der Ansprüche 18 bis 22, wobei die Rotationsgeschwindigkeit bzw. Umfangsgeschwindigkeit einer Trommel ermittelt wird und unter Nutzung des Geschwindigkeitssignals ein Schlupf zwischen der Umfangsgeschwindigkeit der Spule einerseits und der Umfangsgeschwindigkeit der Trommel andererseits ermittelt wird, und insbesondere wobei der Schlupf zwischen der Trommel und der Spule überwacht und mit einem vorgegebenen Soll-Schlupf oder Schlupfprofil verglichen wird, wobei ein Warnsignal ausgegeben und/oder der Spulprozess automatisch korrigiert (manipuliert) und/oder beendet wird, wenn der Schlupf vom Soll-Schlupf oder Schlupfprofil abweicht.

24. Verwendung nach einem der Ansprüche 18 bis 23, wobei der Schlupf zwischen einer Trommel und der Spule beim Hochfahren des Spulprozesses überwacht wird, und insbesondere wobei ein Antrieb auf einen vorgegebenen Soll-Schlupf und/oder ein vorgegebenes Schlupfprofil geregelt wird.

25. Verwendung des Verfahrens oder der Vorrichtung nach einem der Ansprüche 1 bis 14, zum berührungslosen Bestimmen eines Bruchs eines laufenden Fadens oder eines Einklemmens eines laufenden Fadens, insbesondere über Ermittlung einer Geschwindigkeitsänderung.

**Claims**

1. Method for measuring the speed of a yarn (5) with a first and a second coherent light wave (6a, 6b) which are brought to interference, wherein the first light wave (6a) contains light scattered from the yarn (5) and originates from a first light beam (4a) which is projected on the yarn (5), and wherein the second light wave (6b) originates from a second light beam (4b), and wherein the speed of the yarn (5) is determined from the Doppler interference between the light waves (6a, 6b) in a light detector (8), **characterized in that** the second light beam (4b) is guided onto the light detector (8) directly as second light wave (6b) without contacting the yarn (5), wherein the first and the second light beam (4a, 4b) run parallel but mutually shifted.

2.  Method according to claim 1, wherein the first and the second light beam (4a, 4b) do not overlap.

3.  Method according to one of the preceding claims, wherein the first and the second light beam (4b) are generated by projecting an initial light beam (2) from a coherent light source onto an at least partially transparent body (20) and separating it by this body (20) by reflexions.

4.  Method according to claim 3, wherein the first and the second light beam (4a, 4b) are generated by projecting an initial light beam (2) from a coherent light source (1) onto an at least partially transparent body (20) and separating it by this body (20) by reflexions, wherein the reflexions take place at mutually parallel surfaces (12, 13) of the body (20).

5.  Method according to claim 4, wherein the initial light beam (2) enters the body (20) through a first surface (12) and a part of the initial light beam (2) is reflected for generating the first and the second light beam (4a, 4b), wherein thereafter the initial light beam (2) is reflected at a second surface (13) facing the first surface (12) of the body (20) and thereafter leaves the body (20) again through the first surface (12) in order to generate the second or the first light beam (4a, 4b) respectively.

6.  Method according to one of the preceding claims, wherein a section of the yarn (5) is imaged on the light detector (8) by an imaging optics (7) and wherein the second light beam (4b) is guided onto the light detector (8) past the imaging optics (7).

7.  Method according to one of the preceding claims, wherein position and/or length of defects in the yarn is determined from the measured speed.

8.  Method according to one of the preceding claims, wherein the yarn is winded on and wherein the length of the wound yarn is determined from the measured speed.

9.  Method according to one of the preceding claims, wherein the yarn is wound on and wherein a time dependent speed course is recorded from the measured speed.

10.  Method according to claim 9, wherein the yarn is guided, in a winding station, through a cam drum which imposes the yarn a known run which is given by the geometry of the cam drum, and wherein the followed speed course is compared to the known course, particularly in order to check if the yarn is guided correctly in the cam drum.

11.  Method according to one of the preceding claims, wherein an acceleration of the yarn is determined from the measured speeds, and particularly that the acceleration is used as a measure for a yarn traction force.

12.  Method according to one of the preceding claims, wherein the first light beam (4a) is unfocused at the yarn and/or has a diameter of at least 1 mm, particularly at least 2 mm.

13.  Device for measuring the speed of a yarn (5), comprising
    yarn guiding means (10) for guiding the yarn (5) through a target zone (11) of the yarn,
    a coherent light source (1) for generating an initial light beam (2),
    a beam divider arrangement (3) for generating a first and a second light beam (4b) from the initial light beam (2), wherein the beam divider arrangement is formed in such a way that the first light beam (4a) traverses the target zone of the yarn in order to generate there a first light wave (6a) which is scattered by the yarn (5), and
    a light detector (8) for measuring a Doppler interference between the first light wave (6a) and a second light wave (6b) generated from the second light beam (4b),
    wherein the beam divider arrangement (3) is formed in such a way that the second light beam (4b) does not traverse the target zone of the yarn and therefore falls onto the light detector (8) without contact with the yarn (5)
    **characterized in that** the device is formed in such a way that the second light beam (4b) falls directly onto the light detector (8) as second light wave (6b) and **in that** the beam divider arrangement (3) has a transparent body (20) with a first and a second surface (12, 13) which run parallel to each other, wherein the initial light beam (2) is split into the first and the second light beam (4a, 4b) by reflections at the first and the second surface (12, 13), and wherein the first and the second light beam (4a, 4b) run in parallel and offset manner with respect to one another.

14.  Device according to claim 13, wherein an imaging optics (7) is provided in order to image a zone of the yarn (5) onto the light detector (8) and wherein the second light beam (4b) is guided onto the light detector (8) past the imaging optics (7).

15.  Use of the method or the device according to one of the preceding claims for gathering and/or enhancing the yarn quality of a running yarn at a winding position of a textile machine.

**16.** Use according to claim 15, wherein the position and/or length of a defect is gathered by the doppler interference and particularly wherein the yarn is winded back depending on the position and/or the length of a defect in order to cut out the defect.

**17.** Use according to one of the claims 15 or 16, wherein a measure for the hairiness of the yarn is determined from the Doppler interference, and particularly wherein this measure is output and/or compared with a limit hairiness in order to issue a warning signal in case the limit hairiness is exceeded and/or to reduce a winding speed and/or to reduce a yarn traction force and/or to terminate a winding process.

**18.** Use of the method or of the device according to one of the claims 1 to 14 for gathering and/or enhancing the quality of a yarn package obtained by winding the yarn.

**19.** Use according to claim 18, wherein a winded length is determined and/or the winding process is terminated when reaching a predefined target length or a package change is initiated.

**20.** Use according to one of the claims 18 or 19, wherein a course of the speed of the yarn is measured and the course of the speed is subjected to a spectral decomposition in order to obtain a frequency profile which is compared to an upper and/or a lower limit frequency profile, wherein an error is displayed or measures for correcting the winding process are initiated in case of an exceeding of the upper limit frequency profile or an underrun of the lower limit frequency profile.

**21.** Use according to one of the claims 18 to 20, wherein a diameter of the package, particularly an inner and/or an outer diameter of a cone-shaped package is determined from the measured speed by taking into consideration the laying distance, and particularly wherein the package rotation speed is determined and the diameter is determined from the package rotation together with the measured yarn speed.

**22.** Use according to one of the claims 18 to 21, wherein a density of the package is determined from the measured speed, and particularly wherein the density of the package is output and/or a warning signal is issued if the density exceeds a given upper and/or lower density limit value.

**23.** Use according to one of the claims 18 to 22, wherein the rotation speed or the circumferential speed respectively of a drum is determined and a slip between the circumferential speed of the package on the one hand and the circumferential speed of the drum on the other hand is determined by using the speed signal, and particularly wherein the slip between the drum and the package is checked and compared to a given target slip or slip profile, wherein a warning signal is issued and/or the winding process is automatically corrected (manipulated) and/or terminated if the slip deviates from the target slip or slip profile.

**24.** Use according to one of the claims 18 to 23, wherein the slip between a drum and a package is checked during start up of the winding process, and particularly wherein an actuator is regulated to a given target slip and/or a given slip profile.

**25.** Use of the method or of the device according to one of the claims 1 to 14 for the contact-free determination of a rupture of a running yarn or the jamming of a running yarn, particularly by the determination of a speed change.

**Revendications**

**1.** Méthode pour la mesure de la vitesse d'un fil (5), avec une première et une deuxième onde de lumière cohérente (6a, 6b) qui sont mises en interférence, la première onde de lumière (6a) contenant de la lumière dispersée par le fil (5) et provenant d'un premier faisceau de lumière (4a) qui est projeté sur le fil (5), et la deuxième onde de lumière (6b) provenant d'un deuxième faisceau de lumière (4b), et la vitesse du fil (5) étant déterminée dans un détecteur de lumière (8) à l'aide de l'interférence doppler entre les ondes de lumière (6a, 6b),
**caractérisée en ce que** le deuxième faisceau de lumière (4b) est guidé sur le détecteur de lumière (8) directement comme deuxième onde de lumière (6b) sans contact avec le fil (5), le premier et le deuxième faisceau de lumière (4a, 4b) étant parallèles mais décalés l'un par rapport à l'autre.

**2.** Méthode selon la revendication 1, le premier et le deuxième faisceau de lumière (4a, 4b) ne se chevauchant pas.

**3.** Méthode selon l'une des revendications précédentes, le premier et le deuxième faisceau de lumière (4b) étant générés en projetant un faisceau de lumière initial (2) provenant d'une source lumineuse cohérente sur un corps (20) au moins partiellement transparent et le disperser par ce corps (20) à l'aide des réflexions.

**4.** Méthode selon la revendication 3, le premier et le deuxième faisceau de lumière (4a, 4b) étant générés en projetant un faisceau de lumière initial (2) provenant d'une source lumineuse sur un corps (20) au moins partiellement transparent et le disperser par ce corps (20) à l'aide des réflexions, les réflexions ayant lieu à des surfaces (12, 13) du corps (20) mutuellement parallèles.

5. Méthode selon la revendication 4, le faisceau de lumière (2) initial entrant dans le corps (20) à travers d'une première surface (12) et une partie du faisceau de lumière (2) initial étant reflétée afin de générer le premier et le deuxième faisceau de lumière (4a, 4b), le faisceau de lumière (2) initial étant ensuite reflété par une deuxième surface (13) du corps (20) située en face de la première surface (12) et ensuite sortant de nouveau du corps (20) à travers de la première surface (12), afin de générer le deuxième ou bien le premier faisceau de lumière (4a, 4b).

6. Méthode selon l'une des revendications précédentes, une partie du fil (5) étant imagée sur le détecteur de lumière (8) à l'aide d'une optique d'imagerie (7) et le deuxième faisceau de lumière (4b) étant guidé sur le détecteur de lumière (8) à côté de l'optique d'imagerie (7).

7. Méthode selon l'une des revendications précédentes, une position et/ou une longueur des manques dans le fil étant déterminées à partir de la vitesse mesurée.

8. Méthode selon l'une des revendications précédentes, le fil étant bobiné et une longueur du fil bobiné étant déterminée à partir de la vitesse mesurée.

9. Méthode selon l'une des revendications précédentes, le fil étant bobiné et un cours de vitesse dépendant du temps étant enregistré à partir de la vitesse mesurée.

10. Méthode selon la revendication 9, le fil étant guidé, dans une station de bobinage, par un tambour à came qui impose au fil un cours connu, donné par la géométrie du tambour à came, et le cours de vitesse suivi étant comparé avec le cours connu, particulièrement afin de vérifier si le fil est guidé correctement dans le tambour à came.

11. Méthode selon l'une des revendications précédentes, une accélération du fil étant déterminée à partir des vitesses mesurées et particulièrement l'accélération étant utilisée comme mesure pour une force de traction du fil.

12. Méthode selon l'une des revendications précédentes, le premier faisceau de lumière (4a) étant défocalisé au fil et/ou ayant un diamètre d'au moins 1 mm, particulièrement d'au moins 2 mm.

13. Dispositif pour la mesure de la vitesse d'un fil (5), comprenant

des moyens de guidage de fil (10) pour le guidage du fil (5) à travers d'une zone de consigne (11) du fil, une source lumineuse cohérente (1) pour la génération d'un faisceau de lumière initial (2),

un arrangement de diviseur de faisceau (3) pour la génération d'un premier et d'un deuxième faisceau de lumière (4b) à partir du faisceau de lumière initial (2), l'arrangement de diviseur de faisceau étant formé de sorte que le premier faisceau de lumière (4a) traverse la zone voulue du fil afin de générer là-bas une première onde de lumière (6a) dispersée par le fil, et

un détecteur de lumière (8) pour la mesure d'une interférence doppler entre la première onde de lumière (6a) et une deuxième onde de lumière (6b) générée par le deuxième faisceau de lumière (4b), l'arrangement de diviseur de faisceau (3) étant formé de sorte que le deuxième faisceau de lumière (4b) ne traverse pas la zone de consigne du fil et par conséquent rencontre le détecteur de lumière (8) sans contact avec le fil (5) **caractérisé en ce que** le dispositif est formé de sorte que le deuxième faisceau de lumière (4b) rencontre le détecteur de lumière (8) directement comme deuxième onde de lumière (6b) et **en ce que** l'arrangement de diviseur de faisceau (3) a un corps transparent (20) avec une première et une deuxième surface (12, 13) qui sont parallèles l'un par rapport à l'autre, le faisceau de lumière initial (2) étant divisé entre le premier et le deuxième faisceau de lumière (4a, 4b) par des réflexions à la première et à la deuxième surface (12, 13), le premier et le deuxième faisceau (4a, 4b) étant parallèles mais décalés l'un par rapport à l'autre.

14. Dispositif selon la revendication 13, une optique d'imagerie (7) étant prévue afin d'imager une partie du fil (5) sur le détecteur de lumière (8) et le deuxième faisceau de lumière (4b) étant guidé sur le détecteur de lumière (8) à côté de l'optique d'imagerie (7).

15. Utilisation de la méthode ou du dispositif selon l'une des revendications précédentes, pour capter et/ou améliorer la qualité de fil d'un fil défilant dans une position de bobinage d'une machine textile.

16. Utilisation selon la revendication 15, la position et/ou la longueur d'un manque étant captée à l'aide de l'interférence doppler et particulièrement, afin de découper un manque, le fil étant débobiné en dépendance de la position et/ou longueur du manque.

17. Utilisation selon l'une des revendications 15 ou 16, une mesure pour la pilosité du fil étant déterminée à partir de l'interférence doppler, et particulièrement cette mesure étant sortie et/ou étant comparée avec une pilosité limite afin d'emmétré un signal d'avertissement si la pilosité limite est dépassée et/ou de réduire une vitesse de bobinage et/ou de réduire une force de traction du fil et/ou de terminer un processus de bobinage.

18. Utilisation de la méthode ou du dispositif selon l'une des revendications 1 à 14 pour la capture et/ou l'amélioration de la qualité d'une bobine de fil obtenue par le bobinage du fil.

19. Utilisation selon la revendication 18, une longueur bobinée étant déterminée et/ou le processus de bobinage étant terminé lorsqu'une longueur prévue est atteinte ou un changement de bobine est commencé.

20. Utilisation selon l'une des revendications 18 ou 19, un cours de la vitesse du fil étant mesuré et le cours de la vitesse étant soumis à une décomposition spectrale afin d'obtenir un profile de fréquence qui est comparé avec un haut et/ou un bas profile de fréquence limite, une erreur étant affichée ou des mesures pour corriger le processus de bobinage étant prises si le haut profile de fréquence limite est dépassé ou bien le bas profile de fréquence limite n'est pas atteint.

21. Utilisation selon l'une des revendications 18 à 20, un diamètre de la bobine, particulièrement un diamètre intérieur et/ou extérieur d'une bobine conique, étant déterminé à partir de la vitesse mesurée en considérant la levée de pose et particulièrement le nombre de tours de la bobine étant déterminé et le diamètre étant déterminé à partir du nombre de tours de la bobine et de la vitesse mesurée du fil.

22. Utilisation selon l'une des revendications 18 à 21, une densité de la bobine étant déterminée à partir de la vitesse mesurée, et particulièrement la densité de la bobine étant affichée et/ou un signal d'avertissement étant issu si la densité excède et/ou n'atteint pas une valeur de consigne supérieure ou bien une valeur de consigne inférieure de la densité.

23. Utilisation selon l'une des revendications 18 à 22, la vitesse de rotation ou bien la vitesse circonférentielle d'un tambour étant déterminée et un glissement entre la vitesse circonférentielle de la bobine d'un côté et la vitesse circonférentielle du tambour de l'autre côté étant déterminé par l'utilisation du signal de vitesse, et particulièrement le glissement entre le tambour et la bobine étant surveillé et comparé avec un glissement de consigne donné ou un profile de glissement, un signal d'avertissement étant issu et/ou le processus de bobinage étant corrigé (manipulé) automatiquement et/ou terminé si le glissement dévie du glissement de consigne ou du profile de glissement.

sement.

24. Utilisation selon l'une des revendications 18 à 23, le glissement entre un tambour et une bobine étant surveillé pendant le démarrage du processus de bobinage, et particulièrement un propulseur étant réglé à un glissement de consigne donné et/ou un profile de glissement donné.

25. Utilisation de la méthode ou du dispositif selon l'une des revendications 1 à 14, pour la détermination sans contact d'une rupture d'un fil défilant ou d'un coincement d'un fil défilant, particulièrement par la détermination d'un changement de vitesse.

**Fig. 1**

**Fig. 2**

**Fig. 3**

**Fig. 4**

**Fig. 5**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10342383
- US 20040109155 A